# EUROPEAN PATENT APPLICATION

(11) **EP 3 985 092 A1**
(43) Date of publication of application: **20.04.2022**
(21) Application number: 20826268.3
(22) Date of filing: 31.03.2020
(51) Int. Cl.: C12M 1/00, C12M 3/00, C12Q 1/02

(54) **CELL CULTURE SUBSTRATE, METHOD FOR PRODUCING SAID CELL CULTURE SUBSTRATE, AND SCREENING METHOD USING SAID CELL CULTURE SUBSTRATE**

(30) Priority: 17.06.2019 JP 2019111864
(71) Applicant: Toyo Gosei Co., Ltd., Ichikawa-shi, Chiba 272-0012 (JP)
(72) Inventor: JOMURA, Tomoko, Ichikawa-shi, Chiba 272-0012 (JP); AKAHIRA, Yuki, Ichikawa-shi, Chiba 272-0012 (JP); MIYAZAWA, Takashi, Ichikawa-shi, Chiba 272-0012 (JP)
(74) Representative: Strehl Schübel-Hopf & Partner
(86) International application number: PCT/JP2020/014825
(87) International publication number: WO 2020/255529

(57) **Abstract**

A cell culture substrate having a high cell-adhesion portion and a low cell-adhesion portion, wherein; an adhesiveness to a cell of the high cell-adhesion portion and an adhesiveness to a cell of the low cell-adhesion portion are different from each other, the adhesiveness to the cell of the high cell-adhesion portion to cells is higher than the adhesiveness of the low cell-adhesion portion to the cell; and the high cell-adhesion portion has a cell adhesion layer containing two or more kinds of cell adhesion substances on the surface.

## Description

### TECHNICAL FIELD

Some embodiments of the present invention relate to a cell culture substrate, which is expected to have a wide range of applications in the fields of drug discovery, regenerative medicine and the like, a method for manufacturing the same, and a screening method by using the cell culture substrate.

### BACKGROUND ART

In recent years, in the fields of drug screening in drug discovery and regenerative medicine, three-dimensional culture of cells is a culture method that can reflect the in-vivo closer than conventional two-dimensional culture, and thus attention has been drawn to the culture method for conducting highly accurate toxicity tests of a drug and highly accurate screenings thereof.

Various proposals have been made as a cell culture substrate for three-dimensional culture. Of these, the followings have been proposed as cell culture substrates prepared by using photolithography technology. For example, a cell arrangement control tool has been proposed (Patent Document 1), the cell arrangement control tool obtained by exposing a cell non-adhesive hydrophilic polymer having photosensitivity or a cell adhesion hydrophilic polymer film having photosensitivity through a photomask, and then by developing it. Further, a support material for cell culture has been proposed (Patent Document 2), the support material obtained by a method of applying N-isopropylacrylamide on a substrate, of irradiating an electron beam through a metal mask, of washing with water to form a pattern of poly (N-isopropylacrylamide) being a temperature-sensitive polymer, of immersing it in a cell adhesion protein solution to adsorb a cell adhesion protein on a portion not coated with a polymer.

### CITATION LIST

### Patent Literature

Patent Literature 1: Japanese Unexamined Patent Publication Hei3-7576
Patent Literature 2: International Publication WO 01/068799

### SUMMARY OF INVENTION

### Technical Problem

It has been reported that a synthetic polymer, a cell adhesion protein, or the like is applied to a portion to which cells are adhered in a conventional cell culture substrate. However, there is a case that the cell adhesion substance compatible with each cell may differ.

In addition, if the cell culture substrate is sterilized before cell culture, the cell adhesion substance in the cell adhesion portion may be deteriorated by the sterilization treatment.

In view of these circumstances, an object of some embodiments of the invention is, for example, to provide a cell culture substrate having high cell adhesion for a wide range of cells. In addition, an object of another embodiment of the present invention is to provide a cell culture substrate wherein the deterioration of a cell adhesion property is reduced, for example by sterilizing treatment.

Another object of some embodiments of the present invention is to provide, for example, a manufacturing method for the cell culture substrate and a screening method by using the cell culture substrate.

### Solution to Problem

As a result of diligent studies to solve the problems, the present inventors have found that a cell culture substrate having high cell adhesion to a wide range of cells can be obtained by using a specific cell adhesion substance to complete some embodiments of the invention.

Further, the present inventors have found that it is possible to obtain a cell culture substrate having high cell adhesion by using a specific cell adhesion substance, wherein the deterioration of cell adhesion is reduced even by sterilization treatment to complete some embodiments of the present invention.

One embodiment of the cell culture substrate according to the present invention is a cell culture substrate having a high cell-adhesion portion and a low cell-adhesion portion, wherein; an adhesiveness to a cell of the high cell adhesion portion and an adhesiveness of the low cell-adhesion portion is different, the adhesiveness to a cell of the high cell-adhesion portion is higher than the adhesiveness of the low cell-adhesion portion to the cell; and the high cell-adhesion portion has a cell adhesion layer containing two or more kinds of cell adhesion substances on the surface.

One embodiment of the method for manufacturing a cell culture substrate according to the present invention is a method for manufacturing a cell culture substrate having a high cell-adhesion portion and a low cell-adhesion portion, the method including a step (a) of forming the high cell-adhesion portion above at least a part of a base by using a cell adhesion substance, a step (b) of forming a photosensitive composition layer above at least a part of the base by using a photosensitive composition, and a step (c) of exposing and developing the photosensitive composition layer to form a low cell-adhesion portion, wherein the cell adhesion layer having the high cell-adhesion portion contains two or more kinds of cell adhesion substances. Here, the "base" typically means, for example, a member that serves as a base or a raw material and one that has at least one main surface. That is, "above at least a part of a base" typically means above a part of the at least one main surface.

One embodiment of the screening method according to the present invention uses the cell culture substrate.

### Technical Effect

According to some embodiments of the present invention, it is possible to provide a cell culture substrate having high cell adhesion to a wide range of cells. Further, according to another embodiment of the present invention, a cell culture substrate having high cell adhesion, wherein a deterioration of cell adhesion is reduced even by sterilization treatment, a method for manufacturing the cell culture substrate, and a screening method by using the cell culture substrate may be provided.

### BRIEF DESCRIPTION OF DRAWINGS

[FIG.1] FIG. 1 indicates the states in each well of the cell culture substrate of one embodiment according to the present invention after culturing for 6 hours, compared between the states with and without the cell adhesion layer.
[FIG. 2] Fig. 2 indicates the culture states of human breast cancer cells (MCF7) after 15 hours of culturing, in which the cell adhesion of the cell culture substrate was compared between the states with and without γ-ray irradiation.
[FIG. 3] Fig. 3 indicates the culture states of human hepatoma cells (HepG2) after 15 hours of culturing, in which the cell adhesion of the cell culture substrate was compared between the states with and without γ-ray irradiation.

### EMBODIMENT

Hereinafter, typical examples of some embodiments of the present invention will be specifically described, but the present invention is not limited to this.

### <Cell culture substrate>

One embodiment of the present invention is a cell culture substrate having a high cell-adhesion portion and a low cell-adhesion portion, and an adhesiveness to a cell of the high cell-adhesion portion and an adhesiveness to cell of the low cell-adhesion portion are different from each other, wherein the adhesiveness to a cell of the high cell-adhesion portion is higher than that the adhesiveness to a cell of the low cell-adhesion portion, and the high cell-adhesion portion has a cell adhesion layer containing two or more kinds of cell adhesion substances on the surface of the cell culture substrate.

Here, the "low cell-adhesion portion" is a portion having lower adhesion to cells than the "high cell-adhesion portion", and may have weak adhesion to cells or may have no adhesion to cells. From the viewpoint of three-dimensional culture, the low cell-adhesion portion is preferably a cell non-adhesion portion that does not substantially have adhesion to cells. The "non-adhesion portion of the cell" does not mean that the cells do not adhere at all.

Here, the "surface" typically means the outermost surface of the high cell-adhesion portion in contact with the cells, and the presence of the cell adhesion layer on the outermost surface means that it is possible to develop a remarkable difference in adhesiveness to cells between the adherent portion of the high cell-adhesion portion and that of the low cell-adhesion portion. However, in the case that the adhesiveness of the high cell-adhesion portion to the cells becomes higher than the adhesiveness of the low cell-adhesion portion to the cells, it may not necessarily be the outermost surface.

The cell adhesion substance preferably contains at least one kind of extracellular matrix and at least one kind of polyamino acid.

In the cell adhesion substance, the content of the at least one kind of the polyamino acid is preferably less than the content of the extracellular matrix. As a result, it is possible to suppress a decrease or disappearance of the cell adhesion effect of the extracellular matrix due to the sterilization treatment.

In the cell adhesion substance, the content of the at least one kind of extracellular matrix is preferably within the range of 110 to 6000 parts by mass, more preferably within the range of 120 to 2000 parts by mass and further preferably within the range of 130 to 1000 parts by mass with respect to 100 parts by mass of the polyamino acid.

The at least one kind of extracellular matrix is preferably at least one selected from the group consisting of collagen, cellulose, gelatin, vitronectin, fibronectin, matrigel (also referred to as "mouse EHS sarcoma basement membrane matrix"), laminin, proteoglycan, glycosaminoglycan, hyaluronic acid, elastin, cadherin and fibrinogen.

The at least one kind of the polyamino acid is preferably at least one selected from the group consisting of poly-L-lysine, poly-D-lysine, poly-L-ornithine, poly-L-arginine, poly-L-histidine, poly-L-glutamic acid and poly-L-aspartic acid.

The cell adhesion substance is preferably a combination of two or more kinds of the extracellular matrix and one or more kinds of the polyamino acid. In the combination, the total content of the two kinds of the extracellular matrix with respect to one kind of the polyamino acid is preferably within the range of 110 to 6000 parts by mass with respect to 100 parts by mass of the polyamino acid. When two kinds of extracellular matrix are contained, the content ratio of the first extracellular matrix and the second extracellular matrix is preferably within the range of 1:99 to 99: 1, and preferably within the range of 10:90 to 90:10. When three or more kinds of the extracellular matrix are contained, it is preferable to use the same ratio as above.

By setting the content ratio of each component of the cell adhesion substance in the above range, the adhesiveness to a wide range of cells tends to be improved. In addition, the deterioration of cell adhesion tends to be reduced by the sterilization treatment.

The cell adhesion substance is more preferably a combination of two kinds of extracellular matrix and one kind of the polyamino acid. The cell adhesion substance is more preferably a combination of collagen, vitronectin and poly-L-lysine.

In the cell culture substrate of some embodiments of the present invention, it is preferable that the low cell-adhesion portion is formed of a cured product of a photosensitive composition. It is more preferable that the low cell-adhesion portion is a cured product obtained by pattern molding using the photosensitive composition. By obtaining the low cell-adhesion portion by pattern formation using development, the high cell-adhesion portion and the low cell-adhesion portion can be provided in various shapes. The pattern formation is preferably formed on the bottom surface of each of the wells of the base provided with a plurality of wells (concave compartments) serving as regions for culturing cells. The base provided with the wells may have a plurality of wells, and the commercially available multi-plate having the number of wells of 6, 12, 48, 96 or 384 etc. is recited. By providing a plurality of wells, it is possible to culture cells which are different from each other among the wells, if necessary. By culturing cells, one spheroid may be formed in one well, or a plurality of spheroids may be formed for each highly adherent cell portion in the well.

The material of the substrate will be described later.

In the present invention, when the high cell-adhesion portion and the low cell-adhesion portion are obtained by pattern formation such as a development, the shape of the pattern may vary to some extent between the high cell-adhesion portion and the low cell-adhesion portion in the state that both portions may be dispersed to exist on the entire surface of the cell culture substrate, and a repeating pattern or the like can be mentioned as the shape of the pattern. However, it is not always necessary to form a repeating pattern. Examples of the pattern shape recites, for example, sea-island shape, grid shape, stripe shape, concentric shape, radial shape, checkered shape, or a combination of one or more of these, but the pattern shape is not limited thereto.

The size of the pattern shape may be appropriately selected depending on the cells to be cultured. However, typically, for example, when the pattern shape is concentric, the diameter of the high cell- adhesion portion of one cell is preferably within the range of 1 to 1000 µm, more preferably within the range of 10 to 800 µm, further preferably within the range of 30 to 500 µm and particularly preferably within the range of 50 to 300 µm. Regardless of whether the pattern shape is concentric or not, the area of high cell-adhesion portion of one cell is preferably within the range of 0.75 to 800,000 µm², more preferably within the range of 70 to 500,000 µm², and furthermore preferably within the range of 700 to 200,000 µm², and particularly preferably within the range of 1,900 to 72,000 µm².

The distance between the high cell-adhesion portions each other is appropriately selected depending on the cells to be cultured, but it is preferable that the distance is such that the cultured cells in the adjacent high cell-adhesion portions do not bridge with each other. The distance between centers of gravity is preferably 100 µm or more and 9000 µm or less, more preferably 140 µm or more and 4000 µm or less, and further preferably within the range of 160 to 2000 µm, particularly preferably within the range of 180 to 1000 µm based on center of gravity standard.

As a ratio in both portions of the high cell-adhesion portion in the cell culture substrate, within the range of 1 to 50 area% is preferable, within the range of 10 to 40 area % is more preferable. This ratio is more preferably the ratio at the bottom of the well.

Since the cell culture substrate has a pattern shape, it can be preferably used for three-dimensional culture.

Further, the photosensitive composition preferably includes a photosensitive resin having a photosensitive or light absorbing portion represented by the following formula (1). By forming the pattern shape of the cell culture substrate with a cured product of a specific photosensitive composition, it is possible to improve the selectivity of cell adhesion between the high cell-adhesion portion and the low cell-adhesion portion.

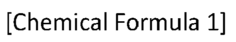

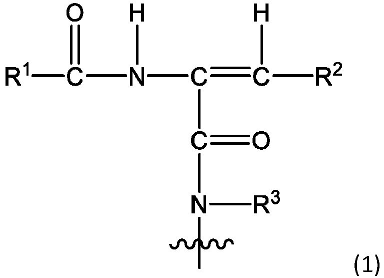

In the formula (1), R¹ is a group selected from the following formula (2). Further, in the formula (1), the lower part of the wavy line is bonded to the polymer main chain.

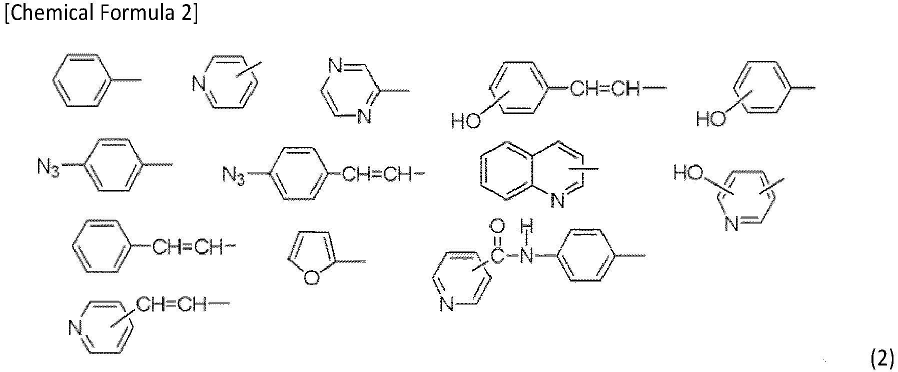

In the formula (1), R² is a group selected from the following formula (3).

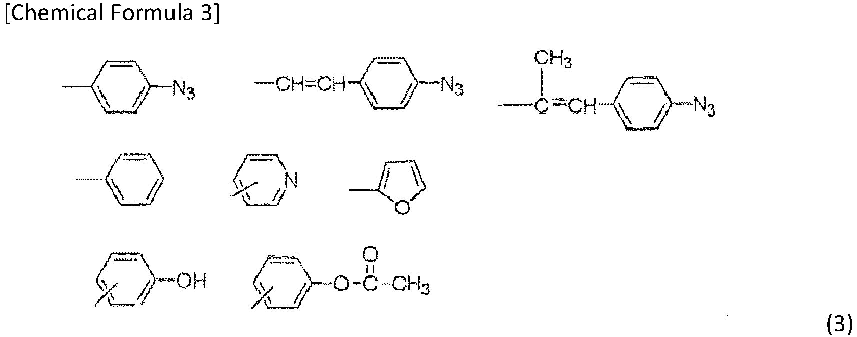

In the formula (1), it is preferable that at least one of R¹ and R² has at least one azide group. In the formula (1), R³ is a hydrogen atom.

The photosensitive resin is bonded to the polymer at the wavy line portion of the photosensitive portion represented by the formula (1). As the photosensitive resin, specifically, it is preferably bonded to any one of the group consisting of either at least one of X and Y of the polymer represented by the following formula (4), the *portion of the polymer having the unit represented by the following formula (5), and the *portion of the polymer having the unit represented by the following formula (6).

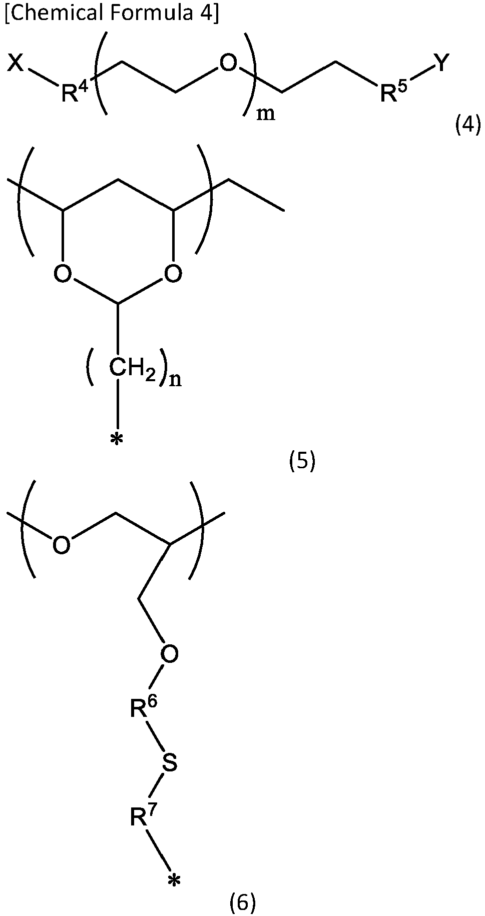

In the formula (4), at least one of X and Y is bonded preferably at the wavy line portion of the photosensitive portion represented by the formula (1), and either one of X or Y may be an amino group. In the formula (4), R⁴ and R⁵, respectively, are preferably an alkylene group having 1 to 10 carbon atoms or a single bond independently. More preferably, it is a methylene group or a single bond. In the case of an alkylene group, at least one methylene group in the alkylene may be substituted with an oxygen atom, and some hydrogens may be substituted with an alkyl group having 1 to 5 carbon atoms. In the formula (4), m is preferably 5 or more.

In the formula (5), n is preferably 1 to 3.

In the formula (6), it is preferable that R ⁶ and R⁷ are independently alkylene groups having 2 to 5 carbon atoms.

When the photosensitive resin is bonded to a polymer having a unit represented by the formula (5), for example, the photosensitive resin can be synthesized as follows. Photosensitive compound is made a pendant with an acetal bond to a polyvinyl alcohol-based polymer having a structure in which at least two repeating units are continuously formed of vinyl alcohol to obtain the photosensitive resin, here, the photosensitive compound is photosensitive portion represented by the formula (1), where the wavy line portion of the photosensitive portion is bonded to one of the groups represented by the following formulas (7) to (8). Specifically, for example, it can be obtained by the method described in International Publication WO2013/153873.

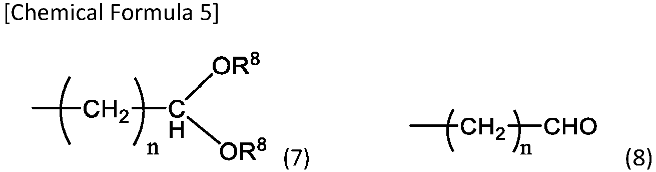

In the formula (7), it is preferable that each R⁸ be independently a linear, branched or cyclic alkyl group having a carbon number of 1 to 6, or an alkylene group having a carbon number of 2 to 6 which is formed together with two R⁸. In the formulas (7) and (8), it is preferable that each n be an integer of 1 to 3 independently.

In some embodiments of the present invention, since the photosensitive resin is water-soluble, the photosensitive composition may contain water in addition to the photosensitive resin. The proportion of the photosensitive resin contained in the photosensitive composition is not particularly limited, but the concentration of the photosensitive resin in the photosensitive composition is preferably within the range of 0.1 to 30% by mass, and more preferably within the range of 4 to 15% by mass.

The photosensitive composition may contain a water-soluble polymer, a water-soluble azide compound, and the like other than the photosensitive resin.

Examples of the water-soluble polymer recite a polyvinyl acetate saponified product, a natural product polymer such as a hydrophilic cellulose derivative and casein, a polymer or copolymer composed of a water-soluble vinyl monomer. Examples of the water-soluble vinyl monomer recite N-vinylformamide, N-vinylacetamide, vinylpyrrolidone, acrylamide, diacetoneacrylamide, N,N-dimethylacrylamide, vinylpyridine, methacrylamide, allylthiourea and the like.

Examples of the water-soluble azide compound may recite 4,4'-diazidestylben-2,2'-disulfonic acid, 4,4'-diazidebenzalacetophenone-2-sulfonic acid, 4,4'-diazidestylben-α-carboxylic acid and alkali metal salts, ammonium salts, organic amine salts and the like thereof.

Furthermore, water-soluble azide compounds described in Japanese Examined Patent Application Sho-50-27404, Japanese Patent Application Laid-Open Sho-50-141403 and Japanese Patent Application Laid-Open Hei-2-204750, Japanese Patent Application Laid-Open Hei-4-26849, Japanese Patent Application Laid-Open Hei-5-11442, Japanese Patent Application Laid-Open Hei-5-113661, Japanese Patent Application Laid-Open Hei-6-239930 and the like can be used suitably.

Furthermore, the photosensitive composition in some embodiments of the present invention may contain, for example, ethylene glycol, sorbitol, a surfactant or the like for improving applying and moisturizing properties.

Further, the photosensitive composition may contain a so-called silane coupling material, which is an adhesion promotor for improving the adhesiveness to the substrate. Examples of the adhesion promotor recite water-soluble adhesion promotors such as N-β (aminoethyl) - aminopropylmethyldimethoxysilane and N-β (aminoethyl) -γ-aminopropyltrimethoxysilane.

Further, the photosensitive composition may contain an antiseptic, an antifoaming agent, a pH adjuster and the like, and may contain a hydrophobic polymer emulsion for improving, for example, film strength, water resistance, base adhesiveness and the like. Examples of the hydrophobic emulsion recite polyvinyl acetate emulsion, polyacrylic acid ester emulsion, urethane emulsion and the like. Pattern formation by using a photosensitive composition including the hydrophobic polymer emulsion can be suitably used, for example, in manufacturing a screen-printing plate.

By using the cell adhesion substance and the photosensitive composition, the selectivity of cell adhesion to high cell-adhesion portion is increased, and the cells tend to be efficiently cultured in the high cell-adhesion portion.

### <Method for manufacturing a cell culture substrate>

One embodiment of the method for manufacturing a cell culture substrate according to the present invention includes a step (a) of forming the high cell-adhesion portion above at least a part of a base by using a cell adhesion substance,
a step (b) of forming a photosensitive composition layer above at least a part of the base by using a photosensitive composition, and
a step (c) of forming the low cell-adhesion portion by exposing and developing the photosensitive composition,
wherein the cell adhesion portion is characterized by having a cell adhesion layer including two or more kinds of cell adhesion substances.

The step (a) may be carried out prior to the step (b), or may be carried out post the step (c).

In the case that the step (a) is carried out prior to the step (b), for example, it is as follows.

One embodiment of the method for manufacturing a cell culture substrate according to the present invention includes;
(1) a step (the step (a)) of forming a cell adhesion layer on at least a part of the base by using a cell adhesion substance,
(2) a step (the step (b)) of forming a photosensitive composition layer on the cell adhesion layer by using the photosensitive composition (the step (b)), and
(3) a step (the step (c)) of obtaining a cell culture substrate by exposing and developing the photosensitive composition layer to form a low cell-adhesion portion,
wherein the high cell-adhesion portion is characterized by being a cell-adhesion layer containing two or more kinds of cell-adhesion substances. By the manufacturing method, the low cell-adhesion portion is formed on the cell adhesion layer in the cell culture substrate to provide the cell culture substrate having a high cell-adhesion portion and a low cell-adhesion portion.

In the case that the step (a) is carried out post the step (c), a cell adhesion layer containing two or more kinds of cell adhesion substances is formed in an area other than the low cell-adhesion portion after patterning a low cell-adhesion portion on at least a part of a base. As a method of forming the cell adhesion layer in the area other than the low cell-adhesion portion, it is presented that the coating liquid for forming the cell adhesion layer described later is applied to the base surface, in which the low cell-adhesion portion is formed in a pattern shape, thereafter the coating liquid for forming the cell adhesion layer is washed with a solution such as water, the solution dissolving cell adhesion substances, to remove the coating liquid for forming the cell adhesion layer on the low cell-adhesion portion, and then the coating liquid for forming the cell adhesion layer remaining on the base is dried to form a cell adhesion portion in a portion other than the low cell-adhesion portion. The formation of the cell adhesion layer is not limited to the application. And here the base surface being both on the low cell-adhesion portion and on the base.

### (1) Step of forming cell adhesion layer (hereinafter, also referred to as "step (a)")

The material of the base used in the step (a) is not particularly limited, and examples thereof recite glass, thermoplastic resin, thermosetting resin, silicon, diamond, metal, ceramic and the like. Further, the shape of the base is not particularly limited, and examples thereof recite a plate shape, a plate shape having a curved surface, a fibrous shape, a substrate having a microporous surface structure, a capillary shape, a tubular shape and the like, but it is not limited thereto. As described above, the base preferably has a plurality of wells (concave compartments), and at least the bottom surface of the wells is preferably colorless and transparent or nearly colorless and transparent in order to observe the behavior of cells during culturing. Of these, glass or transparent plastic can be preferably used as the material, and plate-shaped one can be suitably used.

When the photosensitive composition is exposed to form a pattern on the bottom surface of the well, the side surface of the well may be colored from the viewpoints of suppressing scattering of light from the side surface of the well and the like.

The cell adhesion layer may be formed on a base or a photosensitive composition layer described later.

The cell adhesion substance which is included in the cell adhesion layer is contained in two or more kinds, but it is preferable that the cell adhesion substance contain at least one extracellular matrix and at least one polyamino acid. The cell adhesion substance may be used by adding an aqueous solvent when forming the cell adhesion layer. Examples of the aqueous solvent recite water, alcohols such as ethanol and the like.

In the case that the aqueous solvent in the coating liquid for forming the cell adhesion layer is included, the amount of the aqueous solvent is preferably, but not limited to, adjusted so that the amount of the cell adhesion substance in the coating liquid is within the range of 0.001 to 30% by mass, more preferably within the range of 0.002 to 10% by mass, and further preferably within the range of 0.005 to 1% by mass.

By containing the cell adhesion substance in the coating liquid for forming the cell adhesion layer in the range when forming the cell adhesion layer, the contents of the extracellular matrix and the polyamino acid in the cell adhesion layer can be set in a specific range.

The applying amount of the cell adhesion substance applied to a part of the base is not particularly limited, but it is preferably 300 µL/cm². It is also preferable to wash the applied surface with water after applying and then dry the surface at 28°C to 40°C.

### (2) Step of forming the photosensitive composition layer (hereinafter, also referred to as "step (b)")

The thickness of the photosensitive composition layer when the photosensitive composition is applied onto the base to form the photosensitive composition layer is not particularly limited as long as it can be applied, but a suitable film thickness is 5 nm to 5 µm. When the film thickness is 5 nm or more, it is easy to confirm whether the film is uniformly formed, and when the film thickness is 5 µm or less, it is easy to adjust the viscosity of the solution of the photosensitive composition to be used, and it is preferable in view of applicability.

After the photosensitive composition is applied on the base or the cell adhesion layer, the obtained photosensitive composition layer may be heat-treated if necessary. The heat treatment can be performed, for example, when the solvent or volatile components of the photosensitive composition are evaporated after the application of the photosensitive composition. The conditions of the heat treatment are preferably about 1 minute to 10 hours at 30°C to 150°C, and preferably about 3 minutes to 1 hour at 35°C to 120°C.

### (3) Step of obtaining a cell culture substrate (hereinafter, also referred to as "step (c)")

The entire surface of the photosensitive composition layer may be exposed or a part of surface of photosensitive composition layer may be exposed in desired pattern. When the pattern exposure is performed, a cell culture substrate having an arbitrary pattern shape can be obtained by developing after exposure and removing an unexposed portion.

When the pattern exposure is performed, the exposure may be performed via a mask. As the mask for forming an arbitrary pattern, a mask in which the desired pattern is cut out or a mask composed of only the desired pattern can be used. When the photosensitive resin is a negative type, the type of mask may be designed so that light can pass through the portion to be cured. As the mask, it is preferable that the mask does not transmit light used for exposure as much as possible.

The light source for exposure is not particularly limited as long as the photosensitive group used is capable of being photosensitive. For example, X-rays, electron beams, excimer lasers (F2, ArF, KrF lasers, etc.), high-pressure mercury lamps, and the like can be used as the light source. From these light sources, a wavelength having high photosensitivity efficiency can be appropriately selected. The exposure energy can be appropriately set according to the structure of the photosensitive group and the energy of the light source used. It is usually 0.1 mJ/cm² to 10 J/cm², and preferably about 1 mJ/cm² to 1 J/cm².

When the entire surface is exposed, it may be washed with water after heating if necessary. The heat treatment can be performed, for example, to evaporate excess solvent or volatile components after exposure or to further accelerate the reaction. The conditions of the heat treatment are usually for about 1 minute to 10 hours at 30°C to 150°C, preferably for about 3 minutes to 1 hour at 35°C to 120°C. Further, after the physical properties of the photosensitive resin composition coating film are changed by pattern exposure, heating is performed if necessary, and then development treatment is performed. This heat treatment is also optional and is not particularly conditional, but it is preferably for about 1 minute to 10 hours at 30°C to 150°C, preferably for about 3 minutes to 1 hour at 35°C to 120°C.

The developer for developing is not particularly limited as long as it has a sufficient difference in solubility between the unexposed portion and the exposed portion. As a solvent capable of dissolving the unexposed portion of the photosensitive composition, water or a mixed solution of water and an organic solvent compatible with water can be used. Examples of the organic solvents compatible with water recite ketones such as acetone, lower alcohols such as methanol, acetonitrile, tetrahydrofuran and the like. When these are used, a good pattern with no development residue can be preferably produced. Further, the developer may be a mixed solution as described above, and the concentration thereof is not particularly limited as long as the unexposed portion is dissolved. For example, when the developer is a mixed solution of water and methanol, the concentration of methanol may take any value less than 100% by mass.

Development can be performed by a method of immersing the object to be processed after exposure in the developer, a method of applying or spraying the developer to the object to be processed, or the like. After forming the pattern by development, rinsing, drying steps and the like can be added if necessary.

### (4) Step of sterilizing the cell culture substrate (hereinafter, also referred to as "step (4)")

The method for manufacturing the cell culture substrate may further include a step of sterilizing the cell culture substrate (hereinafter, also referred to as "step (4)").

The step of sterilizing can be performed by any method of sterilization by UV irradiation, sterilization by γ-ray irradiation, sterilization by electron beam irradiation, and sterilization by EOG (ethylene oxide gas).

The irradiation energy for sterilization is usually within the range of about 5 to 40 kGy when using γ-rays, but it is not particularly limited.

In the cell culture substrate of some embodiments of the present invention, since the cell adhesion layer includes a specific cell adhesion substance, deterioration of cell adhesion is unlikely to occur even by a sterilization step.

### <Screening method>

Some embodiments of the present invention relate to a screening method by using the cell culture substrate. Since the cell culture substrate of some embodiments of the present invention has high cell adhesion, operability is good in the culturing step.

### EXAMPLES

Hereinafter, some embodiments of the present invention will be described based on Examples, but the present invention is not limited to these Examples.

### (Example 1)

### <Synthesis of photosensitive resin A and preparation of photosensitive composition>

40 g of polyvinyl alcohol (Nippon Synthetic Chemical Industry Co., Ltd., "Gosenol EG-30": average degree of polymerization 1700) was dissolved in 400 g of water, and 4 g of the photosensitive compound represented by the following formula and 2 g of phosphoric acid were added thereto, and reacted at 60°C for 24 hours. The acetalization reaction rate was 98% as measured by GPC (gel permeation chromatography analysis). From the measurement results, the amount of acetalization was calculated to be an amount corresponding to 0.8 mol% with respect to polyvinyl alcohol. Then, phosphoric acid was removed by treating with an ion exchange resin to obtain a photosensitive liquid containing a photosensitive resin in which a photosensitive compound was bonded to the polyvinyl alcohol with an acetal bond. This photosensitive liquid was diluted with water to prepare a photosensitive composition having a photosensitive resin concentration of 6% by mass.

The photosensitive compound represented by the following formula was synthesized based on the description in International Publication WO2013/153873.

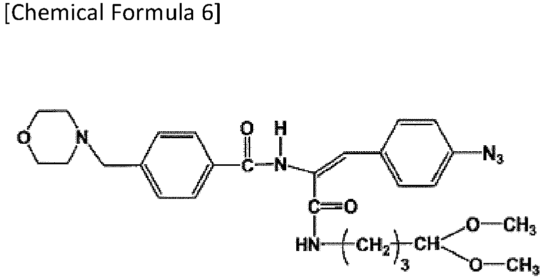

### <Manufacturing method of cell culture substrate 1>

By using water as a solvent, a concentration of collagen was adjusted to 20 µg/mL, a concentration of vitronectin to 1 µg/mL, and a concentration of poly-L-lysine to 10 µg/mL to obtain an aqueous solution of a cell adhesion substance (coating liquid for cell adhesion layer).

A 96-well plate (material: polystyrene, manufactured by Corning Inc., model number: 353219) was prepared, and 100 µL per well of the aqueous solution of the cell culture substance was added and applied. The area of 1 well is 0.32 cm². After applying, it was washed with water and then dried at 40°C for 60 minutes to obtain a substrate on which a cell adhesion layer was formed.

The photosensitive composition obtained above was applied to the substrate on which the cell adhesion layer was formed and dried to obtain a coating film having a film thickness of 1.0 µm. A test mask manufactured by Toppan Printing Co., Ltd. was adhered to this coating film, and the film was exposed for 15 seconds through the mask with an ultra-high pressure mercury lamp (ultraviolet illuminance 5 mW/cm²: measured with an illuminance meter "UV-35" manufactured by ORC Manufacturing Co., Ltd.), then developed with water for 60 seconds and dried. As a result, a circle pattern having a circle diameter of 100 µm and a distance between the centers of gravity of 200 µm with no undeveloped residue was clearly developed.

Then, the plate was irradiated with γ-ray (irradiation condition was about 20 kGy) to obtain a cell culture substrate 1.

### <Cell culture>

A cell culture test was carried out by using the cell culture substrate 1 obtained above. Human breast cancer cells (MCF7), human hepatoma cells (HepG2), and mouse fibroblasts (3T3) were used and cultured each for 10 days. In addition, Dulbecco's modified Eagle's medium containing 10% by mass of fetal bovine serum was used.

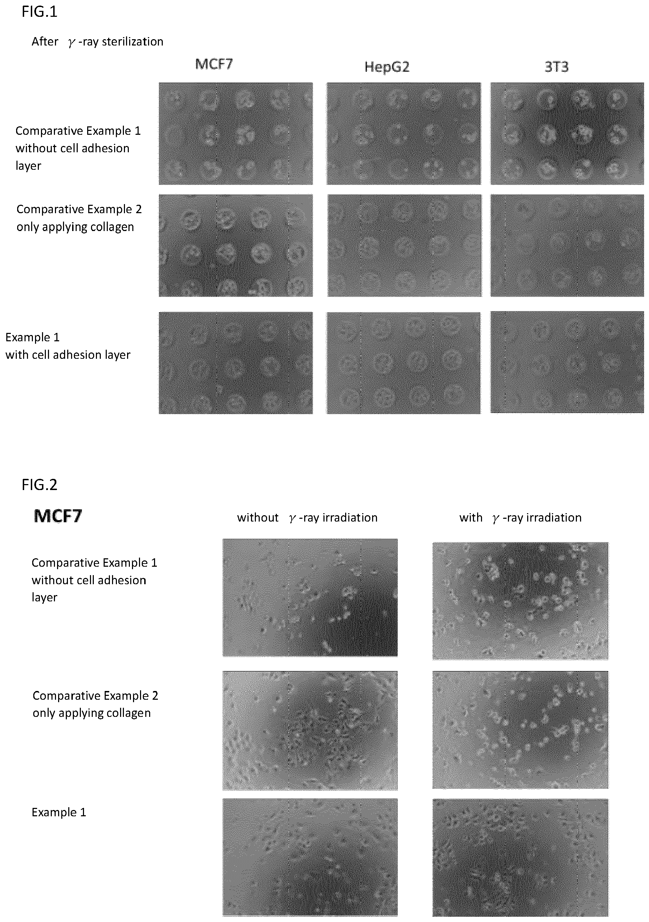

During the period of culturing the three types of cells, the cell culture substrate 1 indicated good cell adhesion to each cell. Each culture state is indicated in FIG. 1. In addition, FIG. 1 is a state in each well after culturing for 6 hours.

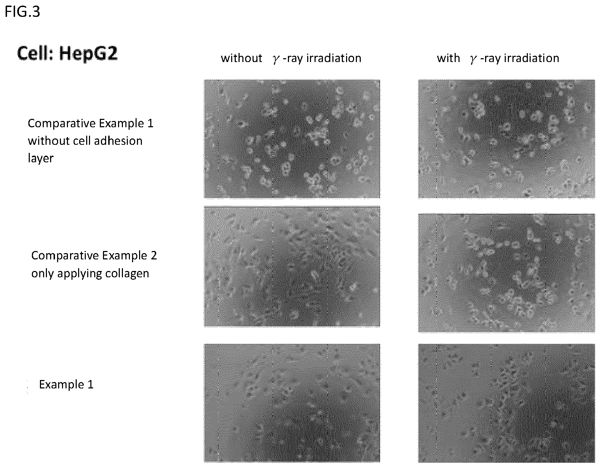

In addition, FIGs. 2 and 3 indicate the results of comparing the cell adhesion of the cell culture substrates with and without γ-ray irradiation. FIG. 2 indicates the culture state of human breast cancer cells (MCF7) after culturing for 15 hours, and FIG. 3 indicates the culture state of human hepatocellular carcinoma cells (HepG2) after culturing for 15 hours.

### <Comparative Examples 1 and 2>

For comparison, the cases of no application of the cell adhesion substance (Comparative Example 1) and the case of using only collagen as the cell adhesion substance (Comparative Example 2) are also illustrated in FIGs. 1 to 3 and Tables 1-2 in the same manner as in Example 1.

The adhesiveness indexes in Tables 1 and 2 are as follows.
⊚ : Adhesion was greatly improved as compared with the case without cell adhesion layer.
○: Adhesion was improved as compared with no cell adhesion layer.
Δ: Adhesion was slightly improved as compared with no cell adhesion layer.
X: No improvement in adhesion was observed as compared with no cell adhesion layer.

**[Table 1]**

| | with/without cell adhesion layer | MCF7 | HepG2 | 3T3 |
|---|---|---|---|---|
| Comparative Example 1 | without adhesion layer | X | X | X |
| Comparative Example 2 | only with collagen | Δ | ○ | x |
| Example 1 | 3 kinds of cell adhesion substances | ○ | ⊚ | ○ |

**[Table 2]**

| | MCF7 | | HepG2 | |
|---|---|---|---|---|
| | without y -ray irradiation | with y -ray irradiation | without y -ray irradiation | with y -ray irradiation |
| Comparative Example 1 | X | X | X | X |
| Comparative Example 2 | ⊚ | Δ | ⊚ | Δ |
| Example 1 | ⊚ | ○ | ⊚ | ○ |

As can be seen from FIGs. 1 to 3, the cell adhesion substrate applied with the specific cell adhesion substance has good cell adhesion without deterioration of the cell adhesion even after the sterilization step. In addition, while there was almost no cell adhesion to the low cell-adhesion portion, cell adhesion to the high cell-adhesion portion was high.

### Industrial Applicability

According to some embodiments of the present invention, it is possible to provide a cell culture substrate having high cell adhesion to various cells, and having reduced deterioration of cell adhesion even when a sterilization treatment is carried out.

## Claims

1. A cell culture substrate having a high cell-adhesion portion and a low cell-adhesion portion,
wherein: anadhesiveness to a cell of the high cell-adhesion portion and an adhesiveness to a cell of the low cell-adhesion portion are different from each other, the adhesiveness to the cell of the high cell-adhesion portion is higher than the adhesiveness of the low cell-adhesion portion to the cell; and
the high cell-adhesion portion has a cell adhesion layer containing two or more kinds of cell adhesion substances on the surface.

2. The cell culture substrate according to claim 1, wherein the cell adhesion substance comprises at least one extracellular matrix and at least one polyamino acid.

3. The cell culture substrate according to claim 2, wherein a content of the at least one polyamino acid is less than a content of the at least one extracellular matrix, in the cell adhesion substance.

4. The cell culture substrate according to claim 3, wherein the content of the at least one extracellular matrix is within the range of 110 to 60,000 parts by mass with respect to 100 parts by mass of the at least one polyamino acid, in the cell adhesion substance.

5. The cell culture substrate according to any one of claims 2 to 4, wherein the at least one extracellular matrix is selected from the group consisting of collagen, cellulose, gelatin, vitronectin, fibronectin, matrigel, laminin, proteoglycan, glycosaminoglycan, hyaluronic acid, elastin, cadherin and fibrinogen.

6. The cell culture substrate according to any one of claims 2 to 5, wherein the polyamino acid is at least one selected from the group consisting of poly-L-lysine, poly-D-lysine, poly-L-ornithine, poly-L-arginine, poly-L-histidine, poly-L-glutamic acid and poly-L-aspartic acid.

7. The cell culture substrate according to any one of claims 2 to 6,
wherein: the at least one extracellular matrix is two or more species selected from the group consisting of collagen, gelatin, vitronectin, fibronectin, matrigel, laminin, proteoglycan, glycosaminoglycan, hyaluronic acid, elastin, cadherin and fibrinogen; and
the at least one of the polyamino acids is at least one species selected from the group consisting of poly-L-lysine, poly-D-lysine, poly-L-ornithine, poly-L-arginine, poly-L-histidine, poly-L-glutamic acid and poly-L-aspartic acid.

8. The cell culture substrate according to any one of claims 1 to 7, wherein the low cell-adhesion portion is formed of a cured product of a photosensitive composition.

9. The cell culture substrate according to claim 8, wherein the photosensitive composition comprises a photosensitive resin having a photosensitive portion represented by the following formula (1) 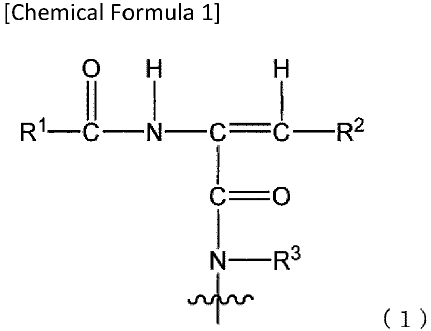 in the formula (1), R¹ is a group selected from the following formula (2), R² is a group selected from the following formula (3), and at least one of R¹ and R² has at least one azide group, 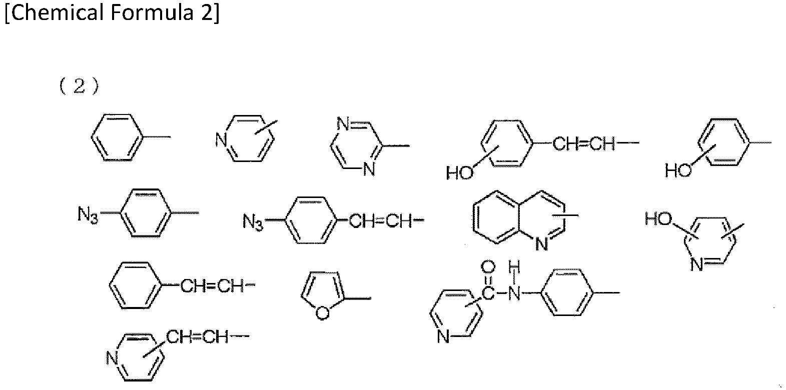 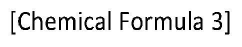 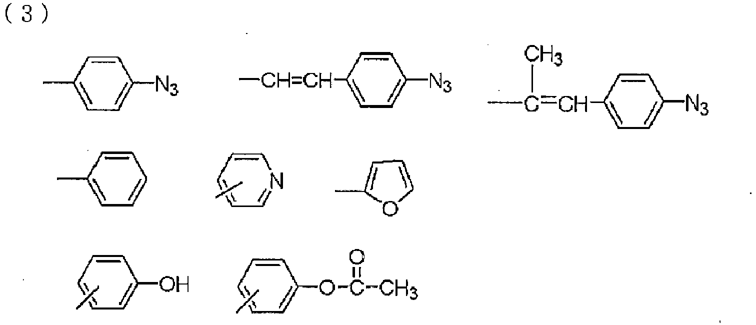 R³ is a hydrogen atom, and a tip of the wavy line is bonded to a polymer main chain.

10. A method for manufacturing the cell culture substrate according to any one of claims 1 to 9, the cell culture substrate comprising a high cell-adhesion portion and a low cell-adhesion portion, wherein the method comprises:
a step (a) of forming the high cell-adhesion portion above at least a part of a base by using a cell adhesion substance;
a step (b) of forming a photosensitive composition layer above at least a part of the base by using a photosensitive composition; and
a step (c) of forming the low cell-adhesion portion by exposing and developing the photosensitive composition,
where the high cell-adhesion portion comprises a cell adhesion layer containing two or more kinds of cell adhesion substances.

11. The method for manufacturing the cell culture substrate according to claim 10, wherein the step (a) is carried out prior to the step (b).

12. The method for manufacturing the cell culture substrate according to claim 10, wherein the step (a) is carried out after the step (c).

13. The method for manufacturing the cell culture substrate according to any one of claims 10 to 12, further comprising a step of sterilizing the cell culture substrate after the step (a) to the step (c).

14. A screening method by using the cell culture substrate according to any one of claims 1 to 9.
